# EUROPEAN PATENT SPECIFICATION

(11) **EP 2 522 358 B1**
(45) Date of publication and mention of the grant of the patent: **09.11.2016**
(21) Application number: 12180141.9
(22) Date of filing: 26.06.2008
(51) Int. Cl.: A61K 38/08, A61K 38/10, C12P 21/06

(54) **Peptides against rotavirus infection**
Peptide gegen Infektionen mit Rotaviren
Peptides contres les infections par le rotavirus

(30) Priority: 27.06.2007 EP 07111189
(43) Date of publication of application: 14.11.2012
(62) Divisional of application: 08774380.3
(73) Proprietor: Laboratorios Ordesa, S.l., 08830 Sant Boi de Llobregat (ES)
(72) Inventor: Rivero Urgell, Montserrat, E-08830 Sant Boi de Llobregat (ES); Fàbrega Sánchez, Joan, E-08006 Barcelona (ES); Moreno Muñoz, José Antonio, E-08222 Terrassa (ES); Bataller Leiva, Esther, E-46008 Valencia (ES); Buesa Gómez, Javier, E-46010 Valencia (ES); Genovés Martínez, Salvador, E-46960 Aldaia (ES); Ramón Vidal, Daniel, E-46183 L'Eliana (ES); Villanueva Roig, Adela, E-46210 Picaña (ES); Casinos Ramo, Beatriz, E-46370 Chiva (ES)
(74) Representative: ZBM Patents - Zea, Barlocci & Markvardsen

(56) References cited:
- EP-A1- 1 359 157
- WO-A2-02/30958
- CLARE D A ET AL: "BIODEFENSE PROPERTIES OF MILK: THE ROLE OF ANTIMICROBIAL PROTEINS AND PEPTIDES", CURRENT PHARMACEUTICAL DESIGN, BENTHAM SCIENCE PUBLISHERS, NL, vol. 9, no. 16, 1 January 2003 (2003-01-01), pages 1239-1255, XP008052495, ISSN: 1381-6128, DOI: 10.2174/1381612033454874
- DATABASE MEDLINE [Online] US NATIONAL LIBRARY OF MEDICINE (NLM), BETHESDA, MD, US 03 October 2001 SUPERTI F ET AL: 'Involvement of bovine lactoferrin metal saturation, sialic acid and protein fragments in the inhibition of rotavirus infection.' Database accession no. NLM11687297 & SUPERTI F ET AL: "Involvement of bovine lactoferrin metal saturation, sialic acid and protein fragments in the inhibition of rotavirus infection.", BIOCHIMICA ET BIOPHYSICA ACTA 3 OCT 2001, vol. 1528, no. 2-3, 3 October 2001 (2001-10-03), pages 107-115, ISSN: 0006-3002

<110> Laboratories Ordesa, S.L.
<120> A novel strain of Bifidobacterium and active peptides against rotavirus infections
<130> P896EP02
<150> EP08774380 (PCT/EP2008/058206)
   <151> 2008-06-26
<160> 19
<170> PatentIn version 3.5
<210> 1
   <211> 11
   <212> PRT
   <213> Artificial
<220>
   <223> peptide from supernatant
<400> 1
   Met His Gln Pro His Gln Pro Leu Pro Pro Thr
   1               5                   10
<210> 2
   <211> 12
   <212> PRT
   <213> Artificial
<220>
   <223> peptide from supernatant
<400> 2
   Met His Gln Pro His Gln Pro Leu Pro Pro Thr Val
   1               5                   10
<210> 3
   <211> 13
   <212> PRT
   <213> Artificial
<220>
   <223> peptide from supernatant
<400> 3
   Met His Gln Pro His Gln Pro Leu Pro Pro Thr Val Met
   1 5 10
<210> 4
   <211> 14
   <212> PRT
   <213> Artificial
<220>
   <223> peptide from supernatant
<400> 4
   Met His Gln Pro His Gln Pro Leu Pro Pro Thr Val Met Phe
   1 5 10
<210> 5
   <211> 12
   <212> PRT
   <213> Artificial
<220>
   <223> peptide from supernatant
<400> 5
   Trp Met His Gln Pro His Gln Pro Leu Pro Pro Thr
   1 5 10
<210> 6
   <211> 13
   <212> PRT
   <213> Artificial
<220>
   <223> peptide from supernatant
<400> 6
   Trp Met His Gln Pro His Gln Pro Leu Pro Pro Thr Val
   1 5 10
<210> 7
   <211> 14
   <212> PRT
   <213> Artificial
<220>
   <223> peptide from supernatant
<400> 7
   Trp Met His Gln Pro His Gln Pro Leu Pro Pro Thr Val Met
   1 5 10
<210> 8
   <211> 15
   <212> PRT
   <213> Artificial
<220>
   <223> peptide from supernatant
<400> 8
   Trp Met His Gln Pro His Gln Pro Leu Pro Pro Thr Val Met Phe
   1               5                   10                  15
<210> 9
   <211> 13
   <212> PRT
   <213> Artificial
<220>
   <223> peptide from supernatant
<400> 9
   Ser Trp Met His Gln Pro His Gln Pro Leu Pro Pro Thr
   1               5                   10
<210> 10
   <211> 14
   <212> PRT
   <213> Artificial
<220>
   <223> peptide from supernatant
<400> 10
   Ser Trp Met His Gln Pro His Gln Pro Leu Pro Pro Thr Val
   1               5                   10
<210> 11
   <211> 15
   <212> PRT
   <213> Artificial
<220>
   <223> peptide from supernatant
<400> 11
   Ser Trp Met His Gln Pro His Gln Pro Leu Pro Pro Thr Val Met
   1               5                   10                  15
<210> 12
   <211> 16
   <212> PRT
   <213> Artificial
<220>
   <223> peptide from supernatant
<400> 12
   Ser Trp Met His Gln Pro His Gln Pro Leu Pro Pro Thr Val Met Phe
   1 5 10 15
<210> 13
   <211> 14
   <212> PRT
   <213> Artificial
<220>
   <223> peptide from supernatant
<400> 13
<210> 14
   <211> 15
   <212> PRT
   <213> Artificial
<220>
   <223> peptide from supernatant
<400> 14
   Gln Ser Trp Met His Gln Pro His Gln Pro Leu Pro Pro Thr Val
   1 5 10 15
<210> 15
   <211> 16
   <212> PRT
   <213> Artificial
<220>
   <223> peptide from supernatant
<400> 15
   Gln Ser Trp Met His Gln Pro His Gln Pro Leu Pro Pro Thr Val Met
   1 5 10 15
<210> 16
   <211> 17
   <212> PRT
   <213> Artificial
<220>
   <223> peptide from supernatant
<400> 16
   Gln Ser Trp Met His Gln Pro His Gln Pro Leu Pro Pro Thr Val Met
   1               5                   10                  15
   Phe
<210> 17
   <211> 20
   <212> DNA
   <213> Artificial
<220>
   <223> primer 616V for 16S rRNA gene
<400> 17
   agagtttgat ymtggctcag 20
<210> 18
   <211> 15
   <212> DNA
   <213> Artificial
<220>
   <223> primer 699R for 16S rRNA gene
<400> 18
   rgggttgcgc tcgtt 15
<210> 19
   <211> 1166
   <212> DNA
   <213> Artificial
<220>
   <223> sequence 16S rRNA
<220>
   <221> misc_feature
   <222> (792)..(792)
   <223> n is a, c, g, or t
<220>
   <221> misc_feature
   <222> (864)..(864)
   <223> n is a, c, g, or t
<220>
   <221> misc_feature
   <222> (885)..(885)
   <223> n is a, c, g, or t
<220>
   <221> misc_feature
   <222> (950)..(950)
   <223> n is a, c, g, or t
<220>
   <221> misc_feature
   <222> (961)..(961)
   <223> n is a, c, g, or t
<220>
   <221> misc_feature
   <222> (993)..(993)
   <223> n is a, c, g, or t
<220>
   <221> misc_feature
   <222> (1006)..(1006)
   <223> n is a, c, g, or t
<220>
   <221> misc_feature
   <222> (1022)..(1022)
   <223> n is a, c, g, or t
<400> 19
   cgaactgaga ccggttttca gggatccgct ccgcgtcgcc gcgtcgcatc ccgttgtacc      60
   ggccattgta gcatgcgtga agccctggac gtaaggggca tgatgatctg acgtcatccc     120
   caccttcctc cgagttaacc ccggcggtcc cccgtgagtt cccggcataa tccgctggca     180
   acacggggcg agggttgcgc tcgttgcggg acttaaccca acatctcacg acacgagctg     240
   acgacgacca tgcaccacct gtgaacccgc cccgaaggga agccgtatct ctacgaccgt     300
   cgggaacatg tcaagcccag gtaaggttct tcgcgttgca tcgaattaat ccgcatgctc     360
   cgccgcttgt gcgggccccc gtcaatttct ttgagtttta gccttgcggc cgtactcccc     420
   aggcgggatg cttaacgcgt tagctccgac acggaacccg tggaacgggc cccacatcca     480
   gcatccaccg tttacggcgt ggactaccag ggtatctaat cctgttcgct ccccacgctt     540
   tcgctcctca gcgtcagtaa cggcccagag acctgccttc gccattggtg ttcttcccga     600
   tatctacaca ttccaccgtt acaccgggaa ttccagtctc ccctaccgca ctcaagcccg     660
   cccgtacccg gcgcggatcc accgttaagc gatggacttt cacaccggac gcgacgaacc     720
   gcctacgagc cctttacgcc caataattcc ggataacgct tgcaccctac gtattaccgc     780
   ggctgctggc angtagttag ccggtgctta ttcaacgggt aaactcactc tcgcttgctc     840
   cccgataaaa gaggtttaca accngaaggc ctccatccct cacgnggggt cgctgcatca     900
   ggcttgcgcc cattgtgcaa tattccccac tgctgcctcc cgtaggagtn tgggccgtat     960
   ntcagtccca atgtggccgg tcgccctctc agnccggcta cccgtngaag ccacggtggg    1020
   cngttacccc gccgtcaagc tgataggacg cgaccccatc ccataccgcg aaagctttcc    1080
   cagaagacca tgcgatcaac tggagcatcc ggcattacca cccgtttcca ggagctattc    1140
   cggtgtatgg ggcaggtcgg tcacgc                                         1166

## Description

The present invention relates to active peptides having anti-rotavirus activity. Thus the invention relates to the use of these agents for the treatment or prophylaxis of diarrhea produced by rotaviruses.

### BACKGROUND ART

Rotavirus belongs to the family *Reoviridae* and was given the name 'rota' because its double capsid looks like a wheel of a cart. Since it was discovered, rotavirus has been confirmed by many epidemiological studies performed worldwide to be a major cause of acute diarrhea in infants and young children. Rotaviruses are divided into 6 different antigenic groups (A-F). Rotaviruses of group A, B and C are found in both human and animals, and rotaviruses of group D, E and F are found in animals only. Rotaviruses of group A are largely distributed among human-derived strains, and subgroups are determined by its inner capsid protein VP6 and serological types are determined by the outside capsid proteins VP7 and VP4. Within group A, 4 subgroups have been distinguished. There are at least 14 G-serotypes, G1 to G14, based on the antigenicity of the VP7 protein, and 12 P-serotypes, based on the antigenic properties of the VP4 protein.

Rotavirus is known as the main cause of acute diarrhea and enteritis in children and in the young animals of cattle, horses, pigs and monkeys. The transmission of the infection occurs mainly by the fecal-oral route. Rotavirus causes frequently an acute watery diarrhea in children under 5 years of age.

This rotavirus-induced diarrhea is the result of perturbances in absorption of intestinal mucosal epithelial cells caused by the viral infection. Once the enterocytes of the small intestine become infected, the virus proliferates in their cytoplasms and causes an impairment in several transport systems (ions and/or solutes and water). Damaged cells suffer desquamation and spout out the virus into the intestinal lumen, so that the virus can be found in feces. When rotavirus replicates, damaged cells of villi are replaced by immature crypt cells not having absorption capability, causing malabsorption of sodium and glucose, which results in diarrhea. In addition, a non-structural rotavirus protein, NSP4, has been identified as a viral enterotoxin. NSP4 increases the intracellular calcium levels and affects the permeability of plasma membranes, causing an efflux of chloride, sodium and water, thereby inducing a secretory diarrhea.

The World Health Organization estimates that five million children under 5 years of age die of diarrhea every year, and 20% of them die of rotavirus induced diarrhea. Rotavirus induced diarrhea prevails in winter and is one of the leading cause of infant mortality in the developing countries. The best measure to prevent the disease is breast-feeding. Since mother's milk includes IgA acting against rotavirus, infantile diarrhea caused by rotavirus can be prevented by breast-feeding.

During the last few years research has focused on the potential use of probiotic agents. Probiotics are defined as live microorganisms which when administered in adequate amounts confer a health benefit on the host. The known benefits of enteral administration of probiotic microorganisms include enhanced host defense to disease improving the properties of the indigenous microbiota and increasing colonization resistance to the harmful microbiota. Probiotics have been suggested to play an important role in the formation or establishment of a well-balanced, indigenous, intestinal microbiota in newborn children or adults receiving high doses of antibiotics. Lactic acid bacteria, especially specific strains of *Lactobacillus* and species of *Streptococcus, Enterococcus* and *Bifidobacterium* genera have been recommended for their use as probiotics.

The bacterial genus *Lactobacillus* has been studied frequently in children with regard to its antidiarrheal properties since the 1960s. Studies published in the world literature have concluded that *Lactobacillus* is indeed safe and effective in treating and preventing infectious diarrhea, antibiotic-associated diarrhea, and diarrhea in children who are very susceptible as a result of poor nutrition, impaired immune status, or frequent exposure to pathogens. Despite these reports, physicians in the United States do not routinely recommend *Lactobacillus,* perhaps believing that its effectiveness has not yet been proven (cf. C.W. Christakis et al., "Lactobacillus therapy for acute infectious diarrhea in children: A meta-analysis" Pediatrics 2002, vol. 109, pp. 678-84). J.M. Saavedra et al., "Feeding of Bifidobacterium bifidum and Streptococcus thermophilus to infants in hospital for prevention of diarrhea and shedding of rotavirus" Lancet 1994, vol. 344, pp. 1046-9, assayed a formula with *Bifidobacterium bifidum* and *Streptococcus thermophilus* with hospitalized children.

A number of studies have shown the efficacy of other probiotic agents, including *Lactobacillus rhamnosus* GG (LGG), in the prevention of diarrhea. Several pediatric clinical trials showed the efficacy of LGG in the treatment of rotavirus gastroenteritis (cf. S. Guandalini et al., "Lactobacillus GG administered in oral rehydration solution to children with acute diarrhea: a multicenter European study", J. Pediatr. Gastroenterol. Nutr. 2000, vol. 30, pp. 54-60).

Other approaches have been proposed for treating diarrhea, for example:
EP 1571213 A1 discloses a new strain of *Bifidobacterium longum* AR81 (KCCM-10492), and an active protein separated from the strain with the sequence of 10 amino acids HLDLAVENT, which have rotavirus-inhibiting activity.

JP 08259597 discloses a protein prepared from the culture of *Bifidobacterium longum* strain SBT 2928 capable of interfering with the adhesion of pathogens *(Escherichia coli, Pseudomonas aeruginosa,* etc.) to asialo-GM1. The protein exhibits a molecular weight 10.4 KDa (gel filtration) or 52 KDa (SDS-PAGE) and pi 5.8. Its N-terminal amino acids are defined as VDPHAIVPSNFDFAFL.

EP 1353681 A1 describes the use of the strain *Bifidobacterium* CNCM-2168 to prevent infection of intestinal cells by rotaviruses.

WO 2003010297 A1 describes probiotic *Bifidobacterium* strains AH208, AH209, AH210, AH211, AH212 or AH214, and their use in the treatment of several diseases.

WO 0042168 A1 describes a strain of *Bifidobacterium* (*Bifidobacterium longum infantis* UCC35624) with significantly immunomodulatory effects, useful in the prophylaxis and/or treatment of undesirable inflammatory activity, especially gastrointestinal inflammatory activity such as inflammatory bowel disease or irritable bowel syndrome.

US 5922375 describes a strain of *Bifidobacterium longum* (JBL 28-1/3300) and its use against diarrhea.

Superti et al., "Involvement of bovine lactoferrin metal saturation, sialic acid and protein fragments in the inhibition of rotavirus infection" BIOCHIMICA ET BIOPHISICA ACTA 3 OCT 2001, vol. 1528, no. 2-3, 3 October 2001, pages 107-115, proposes fragments of bovine lactoferrin for inhibiting rotavirus. The proposed fragments are called fragment 86-258 and fragment 324-329.

On the other hand, fragments of cow milk β-casein are known from the literature for several purposes.

For example document EP1359157 discloses, among several peptides, a peptide of 25 amino acids including SEQ ID NO: 1 of the invention as a metalloproteinase inhibitor. These peptides can be administered by means of food (i.e. dairy products) for providing the inhibitory effect useful for several disorders, such as cancer metastasis, tissue ulceration, arthritis and periodontitis as well as inhibition of osteoclasts. The document proposes a simple procedure for obtaining said inhibitory agents.

Also document WO 02/30958 discloses a decapeptide partially including SEQ ID NO: 1 of the invention. The peptide is proposed for the growth of eukaryotic cells, namely of an insect cell line.

There is an urgent need, both in the developed world and in the developing countries, for products and methods that would be effective in treating infectious diarrhea, and specifically diarrhea caused by rotavirus infection or associated with antibiotic therapy.

### SUMMARY OF THE INVENTION

The problem to be solved by the present invention is the provision of agents with anti-rotavirus activity and thus, effective for the treatment of diarrhea. Thus, inventors have also found active peptides with proven anti-rotavirus activity.

An aspect of the invention relates to the use of a peptide which has a length from 11 to 17 amino acids and an amino acid sequence which comprises SEQ ID NO: 1, wherein said amino acid sequence is selected from the group consisting of SEQ ID NO: 1-16, or a pharmaceutically acceptable salt of the peptide, for the manufacture of a medicament for the therapeutic and/or preventive treatment of diarrhea in an animal including a human, wherein the diarrhea is caused by a rotavirus.

The invention may alternatively be formulated as a method for therapeutic and/or preventive treatment of diarrhea caused by rotavirus, in an animal including a human, comprising administering to said animal in need thereof an effective amount of the peptide of the invention. The treatment of the invention is particularly useful for diarrhea caused by rotavirus infection.

In other aspects the invention relates to the use of a peptide as defined above, or a derivative of said peptide, or a pharmaceutically acceptable salt thereof, for the manufacture of a food product or of a nutritional composition.

The invention can also be formulated as a peptide which has a length from 11 to 17 amino acids and an amino acid sequence which comprises SEQ ID NO: 1, wherein said amino acid sequence is selected from the group consisting of SEQ ID NO: 1-16, or a pharmaceutically acceptable salt of the peptide, for use in the treatment and/or prevention of diarrhea in an animal including a human, wherein the diarrhea is caused by a rotavirus.

These and other objects of the present invention will be further described in the detailed description section that follows, and they are not intended to be limiting of the present invention. Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention.

### DETAILED DESCRIPTION OF PARTICULAR EMBODIMENTS

The detailed characterization of the peptides, and their anti-rotavirus activity, are described in sections further on.

As it is used in the art, the term "microorganism" refers to organisms that are microscopic, and include bacteria, yeasts, fungi, archaea or protists. The term "bacteria" is used herein as in the art, to designate an unicellular microorganism which is prokaryotic, i.e., that lack a membrane-bound nucleus and organelles. The term "probiotic" refers to bacteria in the context of dietary supplements. Generally, the term probiotic refers to dietary supplements containing potentially beneficial bacteria or yeast, with lactic acid bacteria as the most common microbes used. The term "effective amount" as used herein, means an amount of an active ingredient high enough to deliver the desired benefit, but low enough to avoid serious side effects within the scope of medical judgment. The term "pharmaceutically acceptable" as used herein pertains to compounds, materials, compositions, and/or dosage forms which are, within the scope of sound medical judgment, suitable for use in contact with the tissues of a subject (e.g. human) without excessive toxicity, irritation, allergic response, or other problem or complication, commensurate with a reasonable benefit/risk ratio. Each carrier, excipient, etc. must also be "acceptable" in the sense of being compatible with the other ingredients of the formulation. Suitable carriers, excipients, etc. can be found in standard pharmaceutical texts, for example, Remington's Pharmaceutical Sciences, 18th edition, Mack Publishing Company, Easton, Pa., 1990.

The invention relates thus to the use of a peptide which has a length from 11 to 17 amino acids and an amino acid sequence which comprises SEQ ID NO: 1, wherein said amino acid sequence is selected from the group consisting of SEQ ID NO: 1-16, , or a pharmaceutically acceptable salt of the peptide, for the manufacture of a food product, of a nutritional composition or of a medicament for the therapeutic and/or preventive treatment of diarrhea in an animal including a human, wherein the diarrhea is caused by a rotavirus. In particular embodiments, the medicament, the food product or the nutritional composition further comprise a bacterium which has the ability of producing the peptide. The peptide amino acid sequence is selected from the group consisting of SEQ ID NO: 1-16: In a more particular embodiment, the peptide has the amino acid sequence MHQPHQPLPPT (SEQ ID NO: 1) and a molecular weight of 1282.63 Da. In another embodiment, the peptide has 13 amino acids, a molecular weight of 1513 Da, and sequence MHQPHQPLPPTVM (SEQ ID NO: 3). These sequences correspond to fragments of cow milk β-casein. Other peptides with an amino acid sequence comprising SEQ ID NO: 1 can be useful for the purposes of the invention (SEQ ID NO: 2, 4-16).

Peptides of the present invention can be in a composition free of the bacteria of the invention. The composition may comprise acceptable excipients, carriers, diluents, etc. and can be produced in the forms of tablets, pills, disperse preparation, granules, elixirs, suspensions, emulsions, solutions, syrups, etc. The composition can be administered either orally or parenterally and its appropriate dosage will be established by the skilled in the art.

For this purpose, the peptide of the invention can be produced by any method known by the skilled in the art. In this regard, for the preparation of the peptides described above comprises fermenting a substrate which comprises the amino acid sequence SEQ ID NO: 1. The substrate is cow milk β-casein. However, for industrial purposes, it is preferred to produce the peptides of the invention by a direct method, such as chemical peptide synthesis or by *in vitro* protease digestion in laboratory or industrial reactors.

The following sections describe the characterization of the peptides of the invention, and the anti-rotavirus activity of the peptides.

### Antiviral spectrum of the 11-mer synthetic peptide of SEQ ID NO: 1.

The 11-mer peptide of SEQ ID NO: 1 was obtained by chemical synthesis (SPPS; solid phase peptide synthesis). Different clinical isolated strains from rotavirus and specific human virus were used in the study of antiviral spectrum of the 11-mer peptide (MHQPHQPLPPT). In this way, OSU and RF were the strains of rotavirus selected as sialic acid-dependent strains as SA11 done previously (animal origin). Ito and VA70 were the strains of rotavirus chosen as not sialic acid-dependent strains, as Wa done previously (human origin). As well as rotavirus strains, Aichi virus *(Kobuvirus* genera, *Picornaviridae* family, human origin) and an astrovirus from human origin were used for the study. In any case, it was observed that the 11-mer synthetic peptide produced to a greater or lesser extent, inhibition of rotavirus strains, Aichi virus and human astrovirus.

These assays were made following two strategies: Strategy A - infection of cellular line with virus, previously incubated with the 11-mer peptide and Strategy B - incubation of the cellular line with the 11-mer peptide and subsequent infection with virus.

**TABLE 1. Inhibition of sialic acid-dependent rotavirus strains infection by synthetic 11-mer peptide in MA104 cells (previous incubation of cells with peptide and previous incubation of virus with peptide).**

| | peptide/cells | | | | peptide/virus | | | |
|---|---|---|---|---|---|---|---|---|
| | 1 µM | 5 µM | 10 µM | 50 µM | 1 µM | 5 µM | 10 µM | 50 µM |
| SA11 | 32.,64% | 39.67% | 13.22% | 52.89% | 10.67% | 5.62% | 17.98% | 2.81% |
| RF | 0% | 9.20% | 17.82% | 1.15% | 23.02% | 24.82% | 17.27% | 13.31% |
| OSU | 0% | 0% | 0% | 0% | 0% | 0% | 0% | 10.88% |

**TABLE 2. Inhibition of not sialic acid-dependent rotavirus strains infection by synthetic 11-mer peptide in MA104 cells (previous incubation of cells with peptide and previous incubation of virus with peptide).**

| | peptide/cells | | | | peptide/virus | | | |
|---|---|---|---|---|---|---|---|---|
| | 1 µM | 5 µM | 10 µM | 50 µM | 1 µM | 5 µM | 10 µM | 50 µM |
| Wa | 8.22% | 39.27% | 23.29% | 21% | | | | |
| VA70 | 23.33% | 25% | 24.17% | 0% | 0% | 0% | 0% | 0% |
| Ito | 36.67% | 36.67% | 33.33% | 26.67% | 44.44% | 40.74% | 53.7% | 33.33% |

**TABLE 3. Inhibition of sialic acid-dependent rotavirus strains infection by synthetic 11-mer peptide in HT29 cells (previous incubation of cells with peptide and previous incubation of virus with peptide).**

| | peptide/cells | | | | peptide/virus | | | |
|---|---|---|---|---|---|---|---|---|
| | 1 µM | 5 µM | 10 µM | 50 µM | 1 µM | 5 µM | 10 µM | 50 µM |
| SA11 | 32.64% | 39.67% | 13.22% | 52.89% | 10.67% | 5.62% | 17.98% | 2.81% |
| RF | 0% | 9.20% | 17.82% | 1.15% | 23.02% | 24.82% | 17.27% | 13.31% |
| OSU | 0% | 0% | 0% | 0% | 0% | 0% | 0% | 10.88% |

**TABLE 4. Inhibition of not sialic acid-dependent rotavirus strains infection by synthetic 11-mer peptide in HT29 cells (previous incubation of cells with peptide and previous incubation of virus with peptide).**

| | .peptide/cells | | | | peptide/virus | | | |
|---|---|---|---|---|---|---|---|---|
| | 1 µM | 5 µM | 10 µM | 50 µM | 1 µM | 5 µM | 10 µM | 50 µM |
| Wa | 0% | 0% | 0% | 0% | 28.42% | 12.02% | 0.55% | 0% |
| VA70 | 40.91% | 18.18% | 13.64% | 31.82% | 0% | 0% | 0% | 0% |
| Ito | 2.72% | 0% | 21.43% | 6.46% | 27.75% | 26.59% | 27.17% | 11.56% |

**TABLE 5. Inhibition of Aichi virus infection by synthetic 11-mer peptide in Vero cells (previous incubation of cells with peptide and previous incubation of virus with peptide).**

| | peptide/cells | | | | peptide/virus | | | |
|---|---|---|---|---|---|---|---|---|
| | 1 µM | 5 µM | 10 µM | 50 µM | 1 µM | 5 µM | 10 µM | 50 µM |
| Aichi | 0% | 22% | 10% | 34% | 2% | 8% | 0% | 13% |

**TABLE 6. Inhibition of Astrovirus infection by synthetic 11-mer peptide in Caco-2 cells (previous incubation of cells with peptide and previous incubation of virus with peptide).**

| | peptide/cells | | | | peptide/virus | | | |
|---|---|---|---|---|---|---|---|---|
| | 1 µM | 5 µM | 10 µM | 50 µM | 1 µM | 5 µM | 10 µM | 50 µM |
| Astrovirus | 0.37% | 15.50% | 20.84% | 0.38% | 2.63% | 0% | 8.42% | 6.49% |

## Claims

1. Use of a peptide which has a length from 11 to 17 amino acids and an amino acid sequence which comprises SEQ ID NO: 1, wherein said amino acid sequence is selected from the group consisting of SEQ ID NO: 1-16, or a pharmaceutically acceptable salt of the peptide, for the manufacture of a medicament for the therapeutic and/or preventive treatment of diarrhea in an animal including a human, wherein the diarrhea is caused by a rotavirus.

2. The use according to claim 1, wherein the medicament further comprises a bacterium which has the ability of producing the peptide.

3. Use of a peptide which has a length from 11 to 17 amino acids and an amino acid sequence which comprises SEQ ID NO: 1, wherein said amino acid sequence is selected from the group consisting of SEQ ID NO: 1-16, or a pharmaceutically acceptable salt of the peptide, for the manufacture of a food product.

4. The use according to claim 3, wherein the food product further comprises a bacterium which has the ability of producing the peptide.

5. Use of a peptide which has a length from 11 to 17 amino acids and an amino acid sequence which comprises SEQ ID NO: 1, wherein said amino acid sequence is selected from the group consisting of SEQ ID NO: 1-16, or a pharmaceutically acceptable salt of the peptide, for the manufacture of a nutritional composition.

6. The use according to claim 5, wherein the nutritional composition further comprises a bacterium which has the ability of producing the peptide.

7. The use according to any of claims 1-6, wherein the amino acid sequence is SEQ ID NO: 1.

8. The use according to any of claims 1-6, wherein the amino acid sequence is SEQ ID NO: 3.

9. A peptide which has a length from 11 to 17 amino acids and an amino acid sequence which comprises SEQ ID NO: 1, wherein said amino acid sequence is selected from the group consisting of SEQ ID NO: 1-16, or a pharmaceutically acceptable salt of the peptide, for use in the treatment and/or prevention of diarrhea in an animal including a human, wherein the diarrhea is caused by a rotavirus.

## Patentansprüche

1. Verwendung von einem Peptid, welches eine Länge von 11 bis 17 Aminosäuren und eine Aminosäuresequenz hat, welche die SEQ ID NO: 1 umfasst, wobei die Aminosäuresequenz ausgewählt aus der Gruppe bestehend aus SEQ ID NO: 1-16 ist, oder einem pharmazeutisch akzeptablen Salz des Peptids, für die Herstellung eines Arzneimittels für die therapeutische und/oder präventive Behandlung von Durchfall bei einem Tier, einschließlich eines Menschen, wobei der Durchfall durch ein Rotavirus verursacht wird.

2. Die Verwendung nach Anspruch 1, wobei das Arzneimittel weiterhin ein Bakterium umfasst, welches die Fähigkeit hat, das Peptid zu erzeugen.

3. Verwendung von einem Peptid, welches eine Länge von 11 bis 17 Aminosäuren und eine Aminosäuresequenz hat, welche die SEQ ID NO: 1 umfasst, wobei die Aminosäuresequenz ausgewählt aus der Gruppe bestehend aus SEQ ID NO: 1-16 ist, oder einem pharmazeutisch akzeptablen Salz des Peptids, für die Herstellung eines Nahrungsmittels.

4. Die Verwendung nach Anspruch 3, wobei das Nahrungsmittel weiterhin ein Bakterium umfasst, welches die Fähigkeit hat, das Peptid zu erzeugen.

5. Verwendung von einem Peptid, welches eine Länge von 11 bis 17 Aminosäuren und eine Aminosäuresequenz hat, welche die SEQ ID NO: 1 umfasst, wobei die Aminosäuresequenz ausgewählt aus der Gruppe bestehend aus SEQ ID NO: 1-16 ist, oder einem pharmazeutisch akzeptablen Salz des Peptids, für die Herstellung einer Nährstoffzusammensetzung.

6. Die Verwendung nach Anspruch 5, wobei die Nährstoffzusammensetzung weiterhin ein Bakterium umfasst, welches die Fähigkeit hat, das Peptid zu erzeugen.

7. Die Verwendung nach einem der Ansprüche 1-6, wobei die Aminosäuresequenz die SEQ ID NO: 1 ist.

8. Die Verwendung nach einem der Ansprüche 1-6, wobei die Aminosäuresequenz die SEQ ID NO: 3 ist.

9. Ein Peptid, welches eine Länge von 11 bis 17 Aminosäuren und eine Aminosäuresequenz hat, welche die SEQ ID NO: 1 umfasst, wobei die Aminosäuresequenz ausgewählt aus der Gruppe bestehend aus SEQ ID NO: 1-16 ist, oder ein pharmazeutisch akzeptables Salz des Peptids, zur Verwendung in der Behandlung und/oder Prävention von Durchfall bei einem Tier, einschließlich eines Menschen, wobei der Durchfall durch ein Rotavirus verursacht wird.

## Revendications

1. Utilisation d'un peptide qui a une longueur de 11 à 17 acides aminés et une séquence d'acides aminés qui comprend la SEQ ID NO:1, dans laquelle ladite séquence d'acides aminés est choisie dans le groupe constitué des SEQ ID NO:1-16, ou d'un sel pharmaceutiquement acceptable du peptide, pour la fabrication d'un médicament pour le traitement thérapeutique et/ou préventive de la diarrhée chez un animal, y compris un être humain, dans laquelle la diarrhée est causée par un rotavirus.

2. L'utilisation selon la revendication 1, dans laquelle le médicament comprend en outre une bactérie qui a la capacité de produire le peptide.

3. Utilisation d'un peptide qui a une longueur de 11 à 17 acides aminés et une séquence d'acides aminés qui comprend la SEQ ID NO:1, dans laquelle ladite séquence d'acides aminés est choisie dans le groupe constitué des SEQ ID NO:1-16, ou d'un sel pharmaceutiquement acceptable du peptide, pour la fabrication d'un produit alimentaire.

4. L'utilisation selon la revendication 3, dans laquelle le produit alimentaire comprend en outre une bactérie qui a la capacité de produire le peptide.

5. Utilisation d'un peptide qui a une longueur de 11 à 17 acides aminés et une séquence d'acides aminés qui comprend la SEQ ID NO:1, dans laquelle ladite séquence d'acides aminés est choisie dans le groupe constitué des SEQ ID NO:1-16, ou d'un sel pharmaceutiquement acceptable du peptide, pour la fabrication d'une composition nutritionnelle.

6. L'utilisation selon la revendication 5, dans laquelle la composition nutritionnelle comprend en outre une bactérie qui a la capacité de produire le peptide.

7. L'utilisation selon l'une quelconque des revendications 1-6, dans laquelle la séquence d'acides aminés est la SEQ ID NO:1.

8. L'utilisation selon l'une quelconque des revendications 1-6, dans laquelle la séquence d'acides aminés est la SEQ ID NO:3.

9. Un peptide qui a une longueur de 11 à 17 acides aminés et une séquence d'acides aminés qui comprend la SEQ ID NO:1, dans laquelle ladite séquence d'acides aminés est choisie dans le groupe constitué des SEQ ID NO:1-16, ou un sel pharmaceutiquement acceptable du peptide, pour l'utilisation dans le traitement et/ou prévention de la diarrhée chez un animal, y compris un être humain, dans laquelle la diarrhée est causée par un rotavirus.
